(19) Europäisches Patentamt
European Patent Office
Office européen des brevets

(11) Veröffentlichungsnummer: **0 010 298**
**B1**

# (12) EUROPÄISCHE PATENTSCHRIFT

(45) Veröffentlichungstag der Patentschrift: **09.12.81**

(51) Int. Cl.³: **C 07 D 249/08**, A 01 N 43/64

(21) Anmeldenummer: **79104020.7**

(22) Anmeldetag: **18.10.79**

## (54) 1,2,4-Triazol-1-yl-Verbindungen, ihre Herstellung, Fungizide, Verfahren zur Herstellung von Fungiziden und Verfahren zur Bekämpfung von Fungi.

(30) Priorität: **23.10.78 DE 2846127**

(43) Veröffentlichungstag der Anmeldung: **30.04.80 Patentblatt 80/9**

(45) Bekanntmachung des Hinweises auf die Patenterteilung: **09.12.81 Patentblatt 81/49**

(84) Benannte Vertragsstaaten: **AT BE CH DE FR GB IT LU NL SE**

(56) Entgegenhaltungen:
**CH-A-568 712**
**DD-A-117 460**
**DE-A1-2 535 332**
**DE-A1-2 552 967**
**DE-A1-2 713 777**
**US-A-3 912 752**

(73) Patentinhaber: **BASF Aktiengesellschaft, Carl-Bosch-Strasse 38, D-6700 Ludwigshafen (DE)**

(72) Erfinder: **Stubenrauch, Gerd, Dr., Rubensstrasse 36, D-6700 Ludwigshafen (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

**EP 0 010 298 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1,2,4-Triazol-1-yl-Verbindungen, ihre Herstellung, Fungizide, Verfahren zur Herstellung von Fungiziden und Verfahren zur Bekämpfung von Fungiziden

Die vorliegende Erfindung betrifft neue 1,2,4-Triazolderivate, Verfahren zu ihrer Herstellung und ihre Verwendung als Fungizide.

Es ist bekannt, dass der 2-(1′,2′,4′-Triazol-1′-yl)-2-phenylessigsäure-t-butylester fungizide Wirksamkeit hat (DE-OS 26 38 470). Seine Wirkung insbesondere gegenüber Mehltau- und Rostpilzen ist jedoch unzureichend. Daher ist er in der Praxis für den Gebrauch als Pflanzenschutzmittel zur Bekämpfung von Schadpilzen wenig geeignet.

In der CH-PS 568 712, den DE-OS 2 552 967, 2 535 332 und 2 713 777, der US-PS 3 912 752 und der DD-PS 117 460 werden Triazolylderivate mit fungizider Wirkung beschrieben. Ihre Wirksamkeit ist jedoch unbefriedigend.

Es wurden nun neue Verbindungen mit einer besseren fungiziden Wirkung gefunden. Gegenstand der Erfindung sind neue 1,2,4-Triazol-1-yl-Verbindungen der allgemeinen Formel I

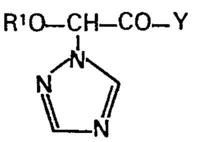

I,

worin

$R^1$ einen durch Cyclohexyl oder Phenyl oder durch 1 bis 3 Halogenatome oder $C_{1-4}$-Alkylgruppen substituierten Phenylrest und

Y $OR^2$ oder 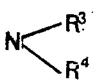 darstellen, worin

$R^2$ einen $C_{3-15}$-Alkinylrest,

$R^3$ ein Wasserstoffatom, einen $C_{1-20}$-Alkyl- oder $C_{3-15}$-Alkenylrest, einen Phenyl- oder Cyanoethylrest und

$R^4$ einen gegebenenfalls durch 1 bis 2 Halogenatome, Trifluormethyl- oder $C_{1-4}$-Alkylgruppen substituierten Phenyl- oder Benzylrest, einen $C_{3-15}$-Alkenyl- oder Alkinylrest, einen Phenylethyl-, Naphthyl- oder Cyanoethylrest bedeuten, sowie deren für Pflanzen verträgliche Salze und Metallkomplexe.

Als pflanzenverträgliche Salze seien beispielsweise die Hydrochloride, -bromide, -sulfate, -nitrate, -phosphate, -oxalate oder -dodecylbenzolsulfonate genannt.

Metallkomplexe sind Verbindungen der Formel VIII

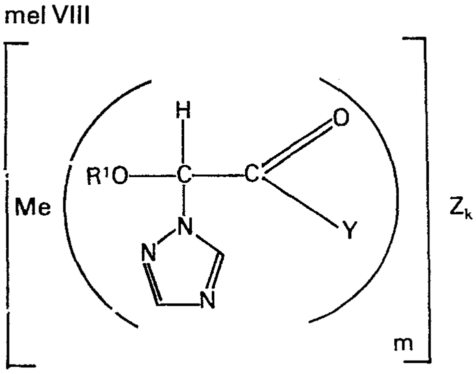

(VIII)

in der $R^1$ und Y die oben angegebene Bedeutung haben und Me ein Metallkation, z.B. von Kupfer, Zink, Zinn, Mangan, Eisen, Cobalt oder Nickel bedeutet, Z für das Anion einer anorganischen Säure steht, z.B. Salzsäure, Schwefelsäure, Salpetersäure, Phosphorsäure oder Bromwasserstoffsäure, und k und m 1, 2, 3 oder 4 bedeuten.

Gegenstand der Erfindung ist weiter ein Verfahren zur Herstellung der 1,2,4-Triazolylverbindungen der allgemeinen Formel I, welches darin besteht, dass man

a) Verbindungen der allgemeinen Formel II

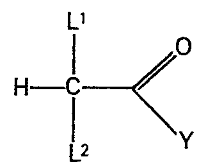

(II)

worin Y die oben aufgeführten Bedeutungen hat und $L^1$ und $L^2$ gleich oder verschieden sind und für nucleophil verdrängbare Abgangsgruppen stehen, mit einer Verbindung der Formel III

$R^1OH$ (III)

oder deren Salzen und 1,2,4-Triazol oder dessen Salzen gegebenenfalls in Gegenwart eines Lösungs- oder Verdünnungsmittels und/oder einer anorganischen oder organischen Base bei Temperaturen zwischen 0° und 180° umsetzt, gegebenenfalls unter Zusatz eines Reaktionsbeschleunigers oder

b) Verbindungen der Formel IV

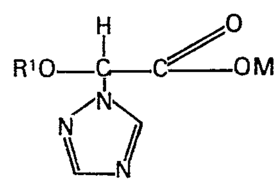

(IV)

worin $R^1$ die oben angegebenen Bedeutungen hat und M für Wasserstoff, ein Äquivalent eines Metallkations oder ein gegebenenfalls substituiertes Ammoniumion steht, mit einer Verbindung der Formel V

HY (V),

worin Y die oben angegebene Bedeutung hat, nach oder unter gleichzeitiger Zugabe von Reagentien, die zur Derivatisierung von Säuren geeignet sind, gegebenenfalls in Gegenwart eines Lösungs- oder Verdünnungsmittels und/oder von anorganischen oder organischen Basen bei Temperaturen zwischen −20° und +180°C gegebenenfalls in Gegenwart eines Reaktionsbeschleunigers umsetzt oder

c) eine Verbindung der Formel IV mit einer Verbindung der Formel VI

$L^3Y$ (VI),

worin Y die oben angegebene Bedeutung hat und $L^3$ für eine nucleophil verdrängbare Abgangsgruppe steht, gegebenenfalls in Gegenwart eines Lösungs- oder Verdünnungsmittels und/oder Säure-bindender Mittel bei Temperaturen zwischen −20 und +180°C gegebenenfalls in Gegenwart eines Reaktionsbeschleunigers umsetzt oder

d) Verbindungen der Formel VII

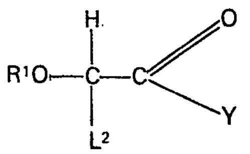

(VII)

worin $R^1$, Y und $L^2$ die oben genannte Bedeutung besitzen mit 1,2,4-Triazol gegebenenfalls in Gegenwart eines Lösungs- oder Verdünnungsmitels und/oder einer anorganischen oder organischen Base bei Temperaturen zwischen −20 und 180°C gegebenenfalls in Gegenwart eines Reaktionsbeschleunigers umsetzt und – falls gewünscht – die gemäss a) bis d) erhaltenen Verbindungen in ihre für Pflanzen verträglichen Salze und Metallkomplexe überführt.

Für die in den Verfahren a) und d) erwähnten nucleophil verdrängbaren Abgangsgruppen $L^1$ und $L^2$ seien beispielsweise genannt: Halogen, wie Chlor, Brom oder Jod; Hydrogensulfat; Hydrogensulfonat; gegebenenfalls substituierte Alkylsulfonyloxy-, Arylsulfonyloxy-, Alkylsulfat-, Phenoxy-, Phenylthio-, Oxonium-, Sulfonium- oder Ammonium-Reste. Geeignete anorganische oder organische Basen, die gegebenenfalls auch als Säure-bindende Mittel in die Reaktion a) eingesetzt werden können, sind beispielsweise Alkali- und Erdalkalihydroxide wie Natriumhydroxid, Kaliumhydroxid, Calciumhydroxid, Alkalicarbonate wie Kalium- oder Natriumcarbonat, Alkalihydride wie Natriumhydrid, Alkali- oder Erdalkalialkoholate wie Natriummethylat, Magnesiumethylat oder Natriumisopropylat oder tertiäre Amine wie Trimethylamin, Triethylamin, N,N-Dimethylanilin, N,N-Dimethylcyclohexylamin, N-Methylpiperidin oder Pyridin, Azole wie 1,2,4-Triazol oder Imidazol. Es können aber auch andere übliche Basen verwendet werden.

Geeignete Salze der Verbindungen der Formel III und des 1,2,4-Triazols sind beispielsweise deren Alkali-, Erdalkali- oder gegebenenfalls substituierte Ammoniumsalze.

Zu den bevorzugten Lösungs- bzw. Verdünnungsmitteln gehören Halogenkohlenwasserstoffe wie beispielsweise Methylenchlorid, Chloroform, 1,2-Dichlorethan, Chlorbenzol; aliphatische oder aromatische Kohlenwasserstoffe wie Cyclohexan, Petrolether, Benzol, Toluol oder Xylole, Alkohole wie Methanol, Ethanol, Isopropanol oder n-Butanol, Ester wie Essigsäureethylester, Amide wie Dimethylformamid, Nitrile wie Acetonitril, Sulfoxide, wie Dimethylsulfoxid, Ketone wie Aceton oder Methylethylketon, Ether wie Diethylether, Tetrahydrofuran oder Dioxan oder entsprechende Gemische. Zweckmässigerweise verwendet man das Lösungs- bzw. Verdünnungsmitel in einer Menge von 100 bis 2000 Gew.-%, vorzugsweise 100 bis 1000 Gew.-%, bezogen auf die Einsatzstoffe II bzw. III.

Als Reaktionsbeschleuniger kommen vorzugsweise Metallhalogenide wie Natriumbromid oder Kaliumiodid, Kronenether, quartäre Ammoniumverbindungen wie Tretrabutylammoniumiodid oder Säuren oder Kombinationen dieser Reaktionsbeschleuniger in Frage.

Die erfindungsgemässe Umsetzung wird im allgemeinen bei Temperaturen zwischen 40 und 150°C in einem Zeitraum von 1 bis 60 Stunden durchgeführt, drucklos oder unter Druck, kontinuierlich oder diskontinuierlich.

Man verfährt im allgemeinen so, dass man auf 1 Mol der Verbindung II jeweils 0,5 bis 2, vorzugsweise 0,9 bis 1,3 Mol der Einsatzstoffe III und 1,2,4-Triazol sowie 1 bis 4 Mol, bevorzugt 1,8 bis 2,3 Mol Base und gegebenenfalls 0,01 bis 0,1 Mol des Reaktionsbeschleunigers einsetzt.

In einer bevorzugten Form des Erfindungsgemässen Verfahrens a) vermischt man die Ausgangsstoffe III und 1,2,4-Triazol in beliebiger Reihenfolge mit einer Base und einem Verdünnungsmittel, gibt dann den Einsatzstoff II und gegebenenfalls einen Reaktionsbeschleuniger zu und hält das Reaktionsgemisch für 0,5 bis 120, vorzugsweise 1 bis 60 Stunden, bei der Reaktionstemperatur, die zwischen 40 und 150°C liegen kann.

Zur Isolierung der erfindungsgemässen Verbindungen wird gegebenenfalls das Verdünnungsmittel entfernt, der Rückstand in einem geeigneten Lösungsmittel aufgenommen und mit Wasser gewaschen, um überschüssige Base sowie nicht umgesetzte Ausgangsstoffe der Formeln III und 1,2,4-Triazol zu entfernen. Die nach dem Abziehen des Lösungsmittels verbleibenden Produkte bedürfen im allgemeinen keiner weiteren Reinigung, können aber nötigenfalls nach bekannten Methoden wie Umkristallisation, Extraktion oder Chromatographie weiter gereinigt werden.

Von den bekannten Reagentien, die zur Derivatisierung von Säuren für das Verfahren b) geeignet sind, seien hier – ohne damit die Erfindung einzuschränken – besipielhaft die folgenden genannt: Protonen- oder Lewissäuren, anorganische oder organische Säurehalogenide wie Thionylchlorid, Phosphorpentachlorid, Acetylchlorid oder Chlorkohlensäureester wie der Ethyl-, tert-Butyl- oder Benzylester, Anhydride wie Keten, Diimide wie N,N′-Dicyclohexylcarbodiimid oder Carbonyl- oder Sulfonyldiazole wie Carbonyldiimidazol, Carbonylditriazol, Sulfonyldiimidazol oder Sulfonylditriazol.

Für das Verfahren c) eignen sich besonders

solche Verbindungen der Formel VI in denen $L^3$ für eine nucleophil verdrängbare Austrittsgruppe wie beispielsweise Halogen wie Chlor, Brom oder Jod, eine gegebenenfalls substituierte Alkyl- oder Arylsulfonyloxygruppe wie ein Mesyloxy-, Trifluormethylsulfonyloxy-, Brosyloxy- oder Tosyloxyrest, Alkylsulfat wie Methylsulfat oder die Diazogruppe steht.

Als bevorzugte Lösungs- oder Verdünnungsmittel und als vorzugsweise einsetzbare Säure-bindende Mittel für die Verfahren b), c) und d) kommen die beim Verfahren a) aufgeführten Verbindungen in Betracht.

Die Durchführung der allgemein beschriebenen Umsetzungen gemäss Verfahren b), c) und d) sowie die Isolierung der erfindungsgemässen Stoffe erfolgt nach bekannten Methoden (vgl. Houben-Weyl, Bd. 8, S. 508 ff und S. 653 ff, Stuttgart [1952] und Bd. 15, 2 Stuttgart [1974]).

Die Ausgangsstoffe der allgemeinen Formel IV können hergestellt werden, indem man eine Verbindung der allgemeinen Formel IX

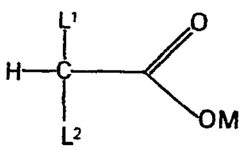

(IX)

worin $L^1$, $L^2$ und M die oben angegebenen Bedeutungen besitzen, mit einer Hydroxyverbindung der allgemeinen Formel III

$R^1OH$ (III)

worin $R^1$ die oben beschriebene Bedeutung hat, oder deren Salzen und 1,2,4-Triazol oder dessen Salzen gegebenenfalls in Gegenwart eines Verdünnungsmittels und/oder Säurebindender Mittel bei Temperaturen zwischen 0 und 180 °C umgesetzt, gegebenenfalls unter Zusatz eines Reaktionsbeschleunigers.

Die Darstellung der Ausgangsstoffe der Formel VII kann nach bekannten Methoden, beispielsweise durch Halogenierung von entsprechenden Aryl- bzw. Heteroaryloxyalkansäurederivaten (vgl. hierzu DE-OS 18 08 034) erfolgen.

Die Verbindungen der Formel I besitzen ein oder mehrere chirale Zentren; reine Isomere können aus den bei der Synthese anfallenden Gemischen nach bekannten Verfahren erhalten werden.

Es können sowohl die reinen Isomere wie auch die Gemische verwendet werden.

Die folgenden Beispiele schildern die Herstellung der neuen Substanzen.

**Herstellung von Ausgangsstoffen**

a) Zu 81,5 Teilen 2,4-Dichlorphenol und 34,5 Teilen 1,2,4-Triazol in 300 Teilen Ethanol gab man 90 Teile einer technischen Natriummethylatlösung (30 Teile Natriummethylat in 100 Teilen methanolischer Lösung) und fügte nach kurzem Rühren 109 Teile Dibromessigsäure zu. Nach 14stündigem Kochen bei Rückflusstemperatur wurde das Lösungsmittel abgezogen und der Rückstand in ca. 1000 Teilen Wasser aufgenommen. Unter Kühlen wurde mit konzentrierter Salzsäure bis pH 1 angesäuert, der ausgefallene Niederschlag abgesaugt und mit Isopropanol gewaschen. Nach dem Trocknen erhielt man 121 Teile 2-(2',4'-Dichlorphenoxy)-2-(1',2',4'-triazol-1'-yl)-essigsäure vom Schmelzpunkt 211 bis 213 °C (Zersetzung).

Zu 1080 Teilen einer technischen Natriummethylatlösung in Methanol (30 Teile Natriummethylat in 100 Teilen Lösung) gab man 771 Teile 4-Chlorphenol und 414 Teile 1,2,4-Triazol. Nach kurzem Nachrühren liess man 774 Teile Dichloressigsäure zulaufen, wobei die Temperatur auf ca. 60 °C anstieg. Man destillierte nun das Methanol ab und liess gleichzeitig Isopropanol so nachlaufen, dass das Reaktionsgemisch ohne Schwierigkeiten gerührt werden konnte. Hatte die Innentemperatur 80 °C erreicht, kochte man 10 Stunden weiter, destillierte dann das Lösungsmittel ab, setzte Eiswasser zu und säuerte mit konzentrierter Salzsäure an. Der ausgefallene Niederschlag wurde abgesaugt, mit Isopropanol nachgewaschen und getrocknet. Man erhielt so 676 Teile (58% der Theorie) 2-(4'-Chlorphenoxy)-2-(1',2',4'-triazol-1'-yl)-essigsäure vom Schmelzpunkt 205 bis 207 °C (Zersetzung).

**Herstellung der Endprodukte**

Beispiel 1

25 Teile 2-(4'-Chlorphenoxy)-2-(1',2',4'-triazol-1'-yl)-essigsäure wurden in 100 Teile Methylenchlorid gegeben und mit 0,5 Teilen Dimethylformamid und 15 Teilen Triethylamin versetzt. Zu dem auf −10 °C abgekühlten Reaktionsgemisch wurden unter Rühren 15 Teile Thionylchlorid getropft. Das Reaktionsgemisch wurde über Nacht bei Raumtemperatur nachgerührt. Dann wurde eine Lösung von 12 Teilen Triethylamin und 12 Teilen Anilin in 20 Teilen Methylenchlorid unter Kühlung bei +10 °C zugetropft. Anschliessend wurde 1 Stunde lang auf Rückflusstemperatur erhitzt. Nach dem Abkühlen wurde der Niederschlag abgesaugt, mit Methylenchlorid gewaschen und die organische Phase eingeengt. Nach Filtration des verbleibenden Öls über Kieselgel mit Methylenchlorid als Elutionsmittel wurden 18 Teile eines einheitlichen Öls erhalten, das nach dem Stehen über Nacht unter Ether kristallisierte.

Man erhielt 15 Teile 2-(4'-Chlorphenoxy)-2-(1',2',4'-triazol-1'-yl)-essigsäureanilid vom Schmelzpunkt 134 bis 136 °C.

Beispiel 2

In 150 Teilen Tetrahydrofuran wurden 23 Teile 2-(4'-Phenylphenoxy)-2-(1',2',4'-triazol-1'-yl)-essigsäure suspendiert und portionsweise mit 13 Teilen N,N'-Carbonyldiimidazol versetzt. Nach einer Stunde Rühren bei Raumtemperatur wurde eine Lösung von 9 Teilen 4-Fluoranilin in 20 Teilen Tetrahydrofuran zugetropft. Nach 12stündigem

Rühren wurde das Reaktionsgemisch in 1000 Teile Eiswasser eingerührt und der ausgefallene Niederschlag abgesaugt und getrocknet.

So wurden 26 Teile 2 (4′-Phenylphenoxy)-2-(1′,2′,4′-triazol-1′-yl)-essigsäure-p-fluoranilid vom Schmelzpunkt 92 bis 96 °C erhalten.

Analog lassen sich beispielsweise folgende Verbindungen herstellen (Temperaturen sind in °C angegeben):

| Nr. | R1 | R2 | R3 | R4 | X | Y | $Fp/n_D^{20}$ |
|---|---|---|---|---|---|---|---|
| 3 | 4-Cl-$C_6H_4$- | H | H | H | O | $OC(CH_3)_2C{\equiv}CH$ | 91–94 |
| 4 | 2,4-DiBr-$C_6H_3$- | H | H | H | O | $OC(CH_3)_2C{\equiv}CH$ | |
| 5 | 2-Br-4-Cl-$C_6H_3$- | H | H | H | O | $OC(CH_3)_2C{\equiv}CH$ | |
| 6 | 2,4-DiCl-$C_6H_3$- | H | H | H | O | $OC(CH_3)_2C{\equiv}CH$ | 60–63 |
| 7 | 2,4-DiCl-$C_6H_3$- | H | H | H | O | $NHC(CH_3)_2C{\equiv}CH$ | 112–115 |
| 8 | 2,4-Di$CH_3C_6H_3$- | H | H | H | O | $NHC(CH_3)_2C{\equiv}CH$ | |
| 9 | 4-Cl-$C_6H_4$- | H | H | H | O | $NHC(CH_3)_2C{\equiv}CH$ | 74–82 |
| 10 | 3-F-$C_6H_4$- | H | H | H | O | $NHC(CH_3)_2CH{\equiv}CH_2$ | |
| 11 | 2,4-DiCl-Cl-$C_6H_3$- | H | H | H | O | $NHC_6H_5$ | 92–95 |
| 12 | 2,4-Di$CH_3$-$C_6H_3$ | H | H | H | O | $NHC_6H_5$ | 114–116 |
| 13 | 2,4-DiCl-$C_6H_3$- | H | H | H | O | NH-2-F-$C_6H_4$ | 99–101 |
| 14 | 4-Br-$C_6H_4$- | H | H | H | O | NH-2-F-$C_6H_4$ | |
| 15 | 4-Cl-$C_6H_4$- | H | H | H | O | NH-3-F-$C_6H_4$ | 102–105 |
| 16 | 2,4-DiCl-$C_6H_3$- | H | H | H | O | NH-3-F-$C_6H_4$ | 80–84 |
| 17 | 3-$CF_3$-$C_6H_4$- | H | H | H | O | NH-3-F-$C_6H_4$ | |
| 18 | 4-Cl-$C_6H_4$- | H | H | H | O | NH-4-$C_6H_4$ | 138–142 |
| 19 | 2,4,5-TriCl-$C_6H_2$- | H | H | H | O | NH-4-F-$C_6H_4$ | 136–140 |
| 20 | 2,4-DiCl-$C_6H_3$- | H | H | H | O | NH-4-F-$C_6H_4$ | 139–142 |
| 21 | 4-Cl-$C_6H_4$- | H | H | H | O | NH-4-Cl-$C_6H_4$ | 146–149 |
| 22 | 2-Br-4Cl-$C_6H_3$- | H | H | H | O | NH-4-Cl-$C_6H_4$ | |
| 23 | 3-F-$C_6H_4$- | H | H | H | O | NH-4-Cl-$C_6H_4$ | |
| 24 | 2,4-DiCl-$C_6H_3$- | H | H | H | O | NH-2,4-DiF-$C_6H_3$ | 122–124 |
| 25 | 3,4-DiCl-$C_6H_3$- | H | H | H | O | NH-3,4-DiF-$C_6H_3$ | |
| 26 | 2,4-Di$CH_3$-$C_6H_3$- | H | H | H | O | NH-3Cl-4F-$C_6H_3$ | |
| 27 | 2,4-DiCl-$C_6H_3$- | H | H | H | O | NH-3,5-DiCl-$C_6H_3$ | 170–173 |
| 28 | 3-$CHF_2$-$CF_2O$-$C_6H_4$- | H | H | H | O | NH-3,5-DiCl-$C_6H_3$ | |
| 29 | 4-$CH_3$-$C_6H_4$- | H | H | H | O | NH-2-$CH_3$-$C_6H_4$ | |
| 30 | 2,4-DiCl-$C_6H_3$- | H | H | H | O | NH-2-i-$C_3H_7$-$C_6H_4$ | 90–94 |
| 31 | 4-Cl-$C_6H_4$- | H | H | H | O | NH-3-tert-$C_4H_9$-$C_6H_4$ | 153–158 |
| 32 | 2,4-DiCl-$C_6H_3$- | H | H | H | O | NH-3-tert-$C_4H_9$-$C_6H_4$ | 154–155 |
| 33 | 4-Cl-$C_6H_4$- | H | H | H | O | NH-3-$CF_3$-$C_6H_4$ | 121–124 |
| 34 | 4-tert-$C_4H_9$-$C_6H_4$- | H | H | H | O | NH-3-$CF_3$-$C_6H_4$ | |
| 35 | 2,4-DiCl-$C_6H_3$- | H | H | H | O | NH-3-$CF_3$-$C_6H_4$ | 115–122 |
| 36 | 4-Cl-$C_6H_4$- | H | H | H | O | $N(CH_3)C_6H_5$ | Öl |
| 37 | 3-F-$C_6H_4$- | H | H | H | S | $N(CH_3)C_6H_5$ | |
| 38 | 4-Cl-$C_6H_4$- | H | H | H | O | $N(CH_3)$-4-Cl-$C_6H_4$ | 125–127 |
| 39 | 2,4-DiCl-$C_3H_3$- | H | H | H | O | NH-$CH_2$-$C_6H_5$ | 72–75 |
| 40 | 4-Cl-$C_6H_4$- | H | H | H | O | $N(CH_3)CH_2C_6H_5$ | 1,5652 |
| 41 | 4-Cl-$C_6H_4$- | H | H | H | O | NH– 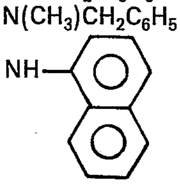 | 144–146 |
| 42 | 2,4-DiCl-$C_6H_3$- | H | H | H | O | 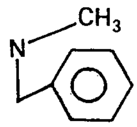 | 106–108 |
| 43 | 4-Cl-$C_6H_4$- | H | H | H | O | NH– 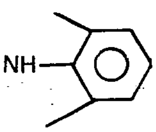 | 152–156 |

| Nr. | $R^1$ | $R^2$ | $R^3$ | $R^4$ | X | Y | $Fp/n_D^{20}$ |
|---|---|---|---|---|---|---|---|
| 44 | 4-Cl-$C_6H_4$- | H | H | H | O | 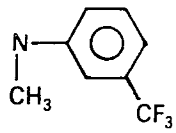 | 1,526 |
| 45 | 4-Cl-$C_6H_4$- | H | H | H | O | 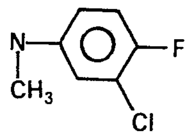 | 1,530 |
| 46 | 4-F-$C_6H_4$- | H | H | H | O | 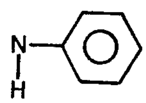 | 102–110 |
| 47 | 2,4-DiCl-$C_6H_3$- | H | H | H | O | $N(CH_3)C_6H_5$ | 145–148 |
| 48 | 2,4-DiCl-$C_6H_3$- | H | H | H | O | N-$CH_2$-$CH=CH_2$ 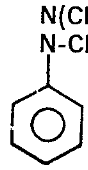 | 76–77 |
| 49 | 3,5-Di$CH_3$-$C_6H_3$- | H | H | H | O | $N(CH_3)C_6H_5$ | 101–103 |
| 50 | 4-tert-$C_4H_9H_4$- | H | H | H | O | NH-4F-$C_6H_4$ | 123–125 |
| 51 | 2,4-DiCl-$C_6H_3$- | H | H | H | O | N-2,4-DiCl-$C_6H_3$ | 109–111 |
| 52 | 4-Cl-$C_6H_4$- | H | H | H | O | $N(CH_2CH_2CN)_2$ | 1,529 |
| 53 | 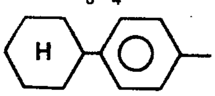 | H | H | H | O | NH-$C_6H_5$ | 120–123 |
| 54 | 4-Cl-$C_6H_4$- | H | H | H | O | N-$C_6H_5$ | 74–76 |
| 55 | 4-Cl-$C_6H_4$- | H | H | H | O | $N(CH_2$-$CH=CH_2)_2$ | 1,5280 |
| 56 | 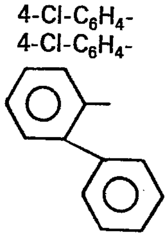 | H | H | H | O | NH-$C_6H_5$ | 110–113 |
| 57 | 2,4,5-TriCl-$C_6H_2$- | H | H | H | O | $NHC(CH_3)_2C{=}CH$ | 176–185 |
| 58 | 4-F-$C_6H_4$- | H | H | H | O | NH-4F-$C_6H_4$ | 127–135 |
| 59 | 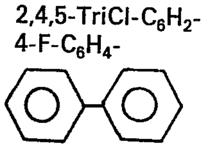 | H | H | H | O | NH 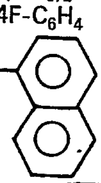 | 105–111 |
| 60 | 2,4,5-TriCl-$C_6H_2$ | H | H | H | O | $NHCH_2$- 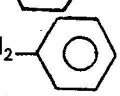 | 168–172 |
| 61 | 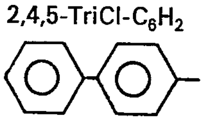 | H | H | H | O | NH-$C_6H_5$ | 158–163 |
| 62 | 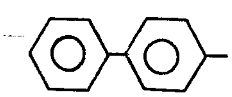 | H | H | H | O | $N(CH_3)C_6H_5$ | 69–74 |
| 63 | 2,4-DiCl-$C_6H_3$– | H | H | H | O | NH-CH-$C_6H_5$ | 102–103 |
| 64 | 2,4-DiCl-$C_6H_3$- | H | H | H | O | NH-$CH_2$2Cl-$C_6H_4$ | 102–105 |
| 65 | 3-tert-$C_4H_9$-$C_6H_3$- | H | H | H | O | $NHC_6H_5$ | 103–106 |
| 66 | 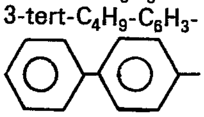 | H | H | H | O | NH-4Cl-$C_6H_4$ | 120–125 |

| Nr. | $R^1$ | $R^2$ | $R^3$ | $R^4$ | X | Y | $Fp/n_D^{20}$ |
|---|---|---|---|---|---|---|---|
| 67 | 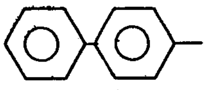 | H | H | H | O | $NHCH_2C_6H_5$ | 71–76 |
| 68 | 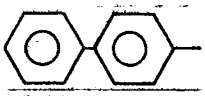 | H | H | H | O | $N(CH_2\text{-}CH{=}CH_2)_2$ | 49–54 |
| 69 | 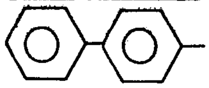 | H | H | H | O | $NH\text{-}3CF_3\text{-}C_6H_4$ | 120–125 |

Die Herstellung der Metallkomplexe wird durch folgendes Beispiel erläutert:

Beispiel 70

17 Teile 2-(4′-Phenylphenoxy)-2-(1′,2′,4′-triazol-1′-yl)-essigsäure-4-fluoranilid wurden in 200 Teilen Ethanol bei 35 °C gelöst und mit einer Lösung von 14 Teilen Kupfer-II-chlorid in 100 Teilen Ethanol versetzt. Nach zweistündigem Rühren wurde auf 15 °C abgekühlt und der ausgefallene Niederschlag abgesaugt.

Es wurden 13 Teile Bis- 2-(4′-phenylphenoxy)-2-(1′,2′,4′-triazol-1′-yl)-essigsäure-4-fluoranilid-kupfer-II-chlorid vom Schmelzpunkt 176 bis 180 °C erhalten.

Analog lassen sich auch die übrigen Verbindungen in ihre Metallkomplexe überführen.

Die erfindungsgemässen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen.

Unter Kulturpflanzen sind in diesem Zusammenhang insbesondere Weizen, Roggen, Gerste, Hafer, Reis, Mais, Baumwolle, Soja, Kaffee, Zuckerrohr, Obst und Zierpflanzen im Gartenbau, sowie Gemüse wie Gurken, Bohnen und Kürbisgewächse zu verstehen.

Die neuen Verbindungen sind insbesondere geeignet zur Bekämpfung folgender Pflanzenkrankheiten:

Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthos poriumarten an Getreide,
Ustilago-Arten an Getreide und Zuckerrohr,
Rhynchosporium secale an Getreide,
Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95 Gew.-% Wirkstoff, vorzugsweise zwischen 0,5 und 90.

Die Aufwandmengen liegen nach Art des gewünschten Effektes zwischen 0,01 und 3, vorzugsweise jedoch zwischen 0,01 und 1 kg Wirkstoff pro Hektar.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen,

Vernebeln, Verstäuben, Verstreuen, Beizen oder Giessen.

Beispiele für solche Zubereitungen sind:

I. Man vermischt 90 Gewichtsteile der Verbindung des Beispiels 6 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 53 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässerige Dispersion, die 0,02 Gew.-% des Wirkstoffs enthält.

III. 20 Gewichtsteile der Verbindung des Beispiels 41 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.-% des Wirkstoffs enthält.

IV. 20 Gewichtsteile der Verbindung des Beispiels 55 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gew.-% des Wirkstoffs enthält.

V. 20 Gewichtsteile der Verbindung des Beispiels 58 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung des Beispiels 30 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung des Beispiels 13 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 1 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gew.-% Wirkstoff enthält.

IX. 20 Teile der Verbindung des Beispiels 2 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden, oder auch mit Dünemitteln vermischt und ausgebracht werden. Beim Vermischen mit Fungiziden erhält man dabei in vielen Fällen eine Vergrösserung des fungiziden Wirkungsspektrums.

Das folgende Beispiel A zeigt die biologische Wirkung der neuen Substanzen. Als Vergleichssubstanz diente der aus der DE-OS 2 638 470 bekannte 2-Phenyl-2-(1′,2′,4′-triazol-1′yl)-essigsäure-t-butylester (N) und die Verbindungen

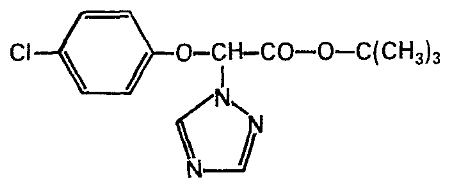

(A)

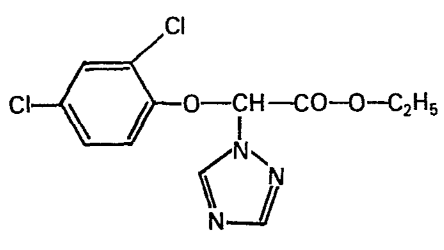

(B)

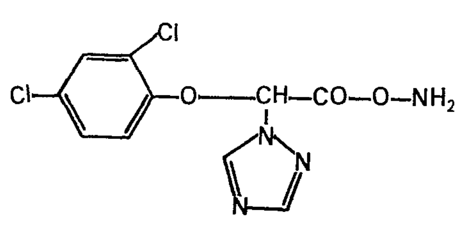

(C)

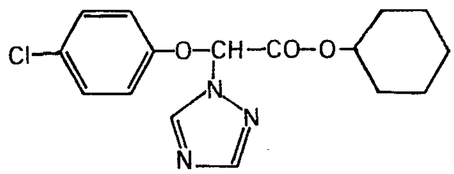

(D)

aus der DE-OS 2 720 654.

Beispiel A

Blätter von in Töpfen gewachsenen Weizensämlingen der Sorte «Caribo» werden mit wässrigen Emulsionen aus 80% (Gew.-%) Wirkstoff und 20% Emulgiermittel besprüht und zwei Tage nach dem Antrocknen des Spritzbelages mit Oidien (Sporen) des Weizenmehltaus (Erysiphe graminis var. tritici) bestäubt. Die Versuchspflanzen werden anschliessend in einer Vegetationshalle bei Temperaturen zwischen 18 und 24 °C aufgestellt. Nach 10 Tagen wird das Ausmass der Mehltaupilzentwicklung ermittelt.

| Substanz des Beispiels | Befall der Blätter nach Spritzung mit ... %iger Wirkstoffbrühe 0,025 | 0,012 | 0,006 |
|---|---|---|---|
| 1 | 0 | 0 | 0 |
| 2 | 0 | 0 | 0 |
| 3 | 0 | 2 | 3 |
| 7 | 0 | 0 | 2 |
| 11 | 0 | 0 | 2 |
| 12 | 1 | 1 | 2 |
| 13 | 0 | 0 | 0 |
| 24 | 0 | 1 | 2 |
| 30 | 0 | 0 | 2–3 |
| 33 | 0 | 0 | 1 |
| 35 | 0 | 2 | 2–3 |
| 38 | 0 | 0 | 2 |
| 39 | 0 | 0 | 0 |
| 41 | 0 | 0 | 2 |
| 42 | 0 | 1 | 2 |
| 43 | 0 | 0 | 0 |
| 44 | 0 | 0 | 3 |
| 45 | 0 | 0 | 0 |
| 46 | 0 | 0 | 0 |
| 48 | 0 | 0 | 0 |
| 52 | 1 | 1 | 1 |
| 53 | 0 | 0 | 0 |
| 54 | 0 | 0 | 0 |
| 55 | 0 | 0 | 0 |
| 56 | 0 | 0 | 2 |
| 58 | 0 | 0 | 0 |
| 59 | 0 | 0 | 0 |
| 60 | 0 | 0 | 0 |
| 61 | 0 | 0 | 0 |
| 62 | 0 | 0 | 0 |
| 63 | 0 | 0 | 0 |
| 64 | 0 | 0 | 0 |
| 65 | 0 | 2 | 2 |
| 66 | 0 | 0 | 2 |
| 67 | 0 | 0 | 0 |
| 68 | 0 | 0 | 0 |
| 69 | 0 | 0 | 1 |
| 70 | 0 | 0 | 0 |
| N | 2 | 3 | 4 |
| C | 4 | 4 | 5 |
| D | 2 | 3 | 4 |
| Kontrolle (unbehandelt) | | 5 | |

0 = kein Pilzwachstum, abgestuft bis 5 = Blätter total befallen

Beispiel B

Blätter von in Töpfen gewachsenen Weizenpflanzen werden mit Sporen des Weizenbraunrostes (Puccinia recondita) künstlich infiziert und 48 Stunden lang bei 20 bis 25 °C in einer wasserdampfgesättigten Kammer aufgestellt. Danach werden die Pflanzen mit wässrigen Spritzbrühen, die in dem Wasser gelöst oder emulgiert eine Mischung aus 80% des zu prüfenden Wirkstoffes und 20% Natriumligninsulfonat enthalten, besprüht und im Gewächshaus bei Temperaturen zwischen 20 und 22 °C und bei 75 bis 80% relativer Luftfeuchtigkeit aufgestellt. Nach 10 Tagen wird das Ausmass der Rostpilzentwicklung beurteilt.

| Substanz des Beispiels | Befall der Blätter nach Spritzung mit ... %iger Wirkstoffbrühe 0,025 | 0,012 | 0,006 |
|---|---|---|---|
| 2 | 0 | 0 | 0 |
| 59 | 0 | 0 | 1 |
| 61 | 0 | 0 | 0 |
| 62 | 0 | 0 | 1 |
| 66 | 0 | 0 | 0 |
| 68 | 0 | 0 | 1 |
| 70 | 0 | 0 | 1 |
| A | 2–3 | 2–3 | 3 |
| B | 2 | 3 | 3 |
| C | 5 | 5 | 5 |
| Kontrolle (unbehandelt) | | 5 | |

0 = kein Pilzwachstum, abgestuft bis 5 = Blätter total befallen

Aus diesen Werten ergibt sich, dass die neuen Verbindungen noch in Konzentration wirken, die mindestens um den Faktor 4 geringer sind als die der Vergleichssubstanzen.

**Patentansprüche für die Vertragsstaaten: BE, CH, DE, FR, GB, IT, LU, NL, SE**

1. 1,2,4-Triazol-1-yl-Verbindungen der allgemeinen Formel I

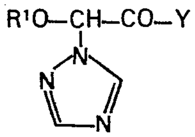 I,

worin

$R^1$ einen durch Cyclohexyl oder Phenyl oder durch 1 bis 3 Halogenatome oder $C_{1-4}$Alkylgruppen substituierten Phenylrest und

Y $OR^2$ oder 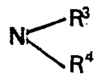 darstellen, worin

$R^2$ einen $C_{3-15}$-Alkinylrest,

$R^3$ ein Wasserstoffatom, einen $C_{1-20}$-Alkyl- oder $C_{3-15}$-Alkenylrest, einen Phenyl- oder Cyanoethylrest und

$R^4$ einen gegebenenfalls durch 1 bis 2 Halogenatome, Trifluormethyl- oder $C_{1-4}$-Alkylgruppen substituierten Phenyl- oder Benzylrest, einen $C_{3-15}$-Alkenyl- oder Alkinylrest, einen Phenylethyl-, Naphthyl- oder Cyanoethylrest bedeuten, sowie deren für Pflanzen verträgliche Salze und Metallkomplexe.

2. Verfahren zur Herstellung der Verbindungen gemäss Anspruch 1, dadurch gekennzeichnet, dass man

a) Verbindungen der Formel II

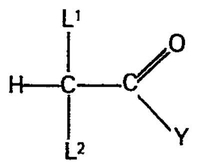 (II)

worin Y die in Anspruch 1 aufgeführten Bedeutungen hat und $L^1$ und $L^2$ gleich oder verschieden sind und für nucleophil verdrängbare Abgangsgruppen stehen, mit einer Verbindung der Formel III

$R^1OH$ (III)

worin $R^1$ die in Anspruch 1 angegebene Bedeutung hat, oder deren Salzen und 1,2,4-Triazol oder dessen Salzen, gegebenenfalls in Gegenwart eines Lösungs- oder Verdünnungsmittels und/oder einer anorganischen oder organischen Base bei Temperaturen zwischen 0 und 180 °C umsetzt, gegebenenfalls unter Zusatz eines Reaktionsbeschleunigers oder

b) Verbindungen der Formel IV

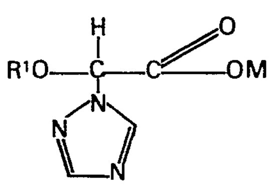 (IV)

worin $R^1$ die in Anspruch 1 angegebene Bedeutung hat und M für Wasserstoff, ein Äquivalent eines Metallkations oder ein gegebenenfalls substituiertes Ammoniumion steht, mit einer Verbindung der Formel V

HY (V)

worin Y die in Anspruch 1 angegebene Bedeutung hat, nach oder unter gleichzeitiger Zugabe von Reagentien, die zur Derivatisierung von Säuren geeignet sind, gegebenenfalls in Gegenwart eines Lösungs- oder Verdünnungsmittels und/oder von anorganischen oder organischen Basen bei Temperaturen zwischen −20° und +180 °C gegebenenfalls in Gegenwart eines Reaktionsbeschleunigers umsetzt oder

c) eine Verbindung der Formel IV mit einer Verbindung der Formel VI

$L^3Y$ (VI)

worin Y die in Anspruch 1 angegebene Bedeutung hat und $L^3$ für eine nucleophil verdrängbare Abgangsgruppe steht, gegebenenfalls in Gegenwart eines Lösungs- oder Verdünnungsmittels und/oder Säure-bindender Mittel bei Temperaturen zwischen −20 und +180 °C gegebenenfalls in Gegenwart eines Reaktionsbeschleunigers umsetzt oder

d) Verbindungen der Formel VII

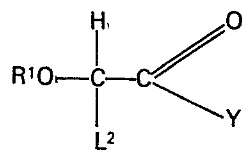 (VII)

worin $R^1$, Y und $L^2$ die oben genannte Bedeutung besitzen mit 1,2,4-Triazol gegebenenfalls in Gegenwart eines Lösungs- oder Verdünnungsmittels und/oder einer anorganischen oder organischen Base bei Temperaturen zwischen −20 und 180 °C gegebenenfalls in Gegenwart eines Reaktionsbeschleunigers umsetzt und – falls gewünscht – die gemäss a) bis d) erhaltenen Verbindungen in ihre für Pflanzen verträglichen Salze und Metallkomplexe überführt.

3. Fungizid, enthaltend mindestens eine Verbindung gemäss Anspruch 1.

4. Fungizid, enthaltend mindestens eine Verbindung gemäss Anspruch 1 und einen festen oder flüssigen Trägerstoff.

5. Verfahren zur Bekämpfung von Fungi, dadurch gekennzeichnet, dass man mindestens eine 1,2,4-Triazol-l-yl-Verbindung gemäss Anspruch 1 auf diese einwirken lässt.

6. Verfahren zur Herstellung von Fungiziden, dadurch gekennzeichnet, dass man 1,2,4-Triazol-1-yl-Verbindungen der Formel I gemäss Anspruch 1 mit festen oder flüssigen Trägerstoffen mischt.

7. Verfahren zur vorbeugenden Bekämpfung von Fungi, dadurch gekennzeichnet, dass man mindestens ein Fungizid gemäss Anspruch 1 auf

durch Fungi-Befall bedrohte Flächen, Pflanzen oder Saatgüter einwirken lässt.

8. Verbindung gemäss Anspruch 1, nämlich
2-(1′,2′,4′-Triazol-1′-yl-)-2-(4′-chlorphenoxy)-essigsäureanilid,
2-(1′,2′,4′-Triazol-1′-yl-)-2-(4′-phenylphenoxy)-essigsäure-p-fluoranilid,
2-(1′,2′,4′-Triazol-1′-yl)-2-(4′(3″-fluorphenylazo)-phenoxy)-essigsäureanilid,
2-(1′,2′,4′-Triazolyl-1′-yl)-2-(4′-chlorphenoxy)-essigsäure-α-naphthylamid.

9. Fungizid, enthaltend eine Verbindung gemäss Anspruch 8.

**Patentansprüche für den Vertragsstaat: AT**

1. Fungizid enthaltend eine 1,2,4-Triazol-1-yl-Verbindung der allgemeinen Formel I

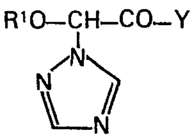
I,

worin
$R^1$ einen durch Cyclohexyl oder Phenyl oder durch 1 bis 3 Halogenatome oder $C_{1-4}$-Alkylgruppen substituierten Phenylrest und

Y $OR^2$ oder 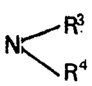 darstellen, worin

$R^2$ einen $C_{3-15}$-Alkinylrest,
$R^3$ einen Wasserstoffatom, einen $C_{1-20}$-Alkyl- oder $C_{3-15}$-Alkenylrest, einen Phenyl- oder Cyanoethylrest und
$R^4$ einen gegebenenfalls durch 1 bis 2 Halogenatome, Trifluormethyl- oder $C_{1-4}$-Alkylgruppen substituierten Phenyl- oder Benzylrest, einen $C_{3-15}$-Alkenyl- oder Alkinylrest, einen Phenylethyl-, Naphthyl- oder Cyanoethylrest bedeuten, sowie deren für Pflanzen verträgliche Salze und Metallkomplexe.

2. Verfahren zur Herstellung der Verbindungen wie im Anspruch 1 definiert, dadurch gekennzeichnet, dass man

a) Verbindungen der Formel II

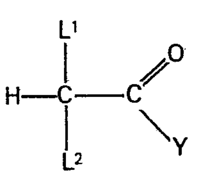
(II)

worin Y die in Anspruch 1 aufgeführten Bedeutungen hat und $L^1$ und $L^2$ gleich oder verschieden sind und für nucleophil verdrängbare Abgangsgruppen stehen, mit einer Verbindung der Formel III

$R^1OH$ (III)

worin $R^1$ die in Anspruch 1 angegebene Bedeutung hat, oder deren Salzen und 1,2,4-Triazol oder dessen Salzen, gegebenenfalls in Gegenwart eines Lösungs- und Verdünnungsmittels und/oder einer anorganischen oder organischen Base bei Temperaturen zwischen 0 und 180 °C umsetzt, gegebenenfalls unter Zusatz eines Reaktionsbeschleunigers oder

b) Verbindungen der Formel IV

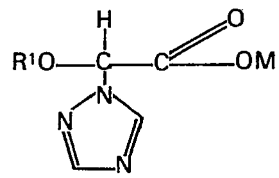
(IV)

worin $R^1$ die in Anspruch 1 angegebene Bedeutung und M für Wasserstoff, ein Äquivalent eines Metallkations oder ein gegebenenfalls substituiertes Ammonium steht, mit einer Verbindung der Formel V

HY (V)

worin Y die in Anspruch 1 angegebene Bedeutung hat, nach oder unter gleichzeitiger Zugabe von Reagentien, die zur Derivatisierung von Säuren geeignet sind, gegebenenfalls in Gegenwart eines Lösungs- oder Verdünnungsmittels und/oder von anorganischen oder organischen Basen bei Temperaturen zwischen −20° und +180 °C gegebenenfalls in Gegenwart eines Reaktionsbeschleunigers umsetzt oder

c) eine Verbindung der Formel IV mit einer Verbindung der Formel VI

$L^3Y$ (VI),

worin Y die in Anspruch 1 angegebene Bedeutung hat und $L^3$ für eine nucleophil verdrängbare Abgangsgruppe steht, gegebenenfalls in Gegenwart eines Lösungs- oder Verdünnungsmittels und/oder Säure-bindender Mittel bei Temperaturen zwischen −20 und +180 °C gegebenenfalls in Gegenwart eines Reaktionsbeschleunigers umsetzt oder

d) Verbindungen der Formel VII

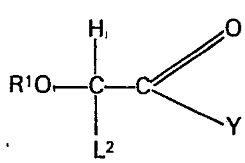
(VII)

worin $R^1$, Y und $L^2$ die oben genannte Bedeutung besitzen mit 1,2,4-Triazol gegebenenfalls in Gegenwart eines Lösungs- oder Verdünnungsmittels und/oder einer anorganischen oder organischen Base bei Temperaturen zwischen −20 und 180 °C gegebenenfalls in Gegenwart eines Reaktionsbeschleuniger umsetzt und – falls gewünscht – die gemäss a) bis d) erhaltenen Verbindungen in ihre für Pflanzen verträglichen Salze und Metallkomplexe überführt.

3. Fungizid, enthaltend mindestens eine Verbindung wie im Anspruch 1 definiert und einen festen oder flüssigen Trägerstoff.

4. Verfahren zur Bekämpfung von Fungi, dadurch gekennzeichnet, dass man mindestens eine 1,2,4-Triazol-1-yl-Verbindung wie im Anspruch 1 definiert auf diese einwirken lässt.

5. Verfahren zur Herstellung von Fungiziden, dadurch gekennzeichnet, dass man 1,2,4-Triazol-1-yl-Verbindungen wie im Anspruch 1 definiert mit festen oder flüssigen Trägerstoffen mischt.

6. Verfahren zur vorbeugenden Bekämpfung von Fungi, dadurch gekennzeichnet, dass man mindestens ein Fungizid gemäss Anspruch 1 auf durch Fungi-Befall bedrohte Flächen, Pflanzen oder Saatgüter einwirken lässt.

7. Fungizid gemäss Anspruch 1, enthaltend
2-(1',2',4'-Triazol-1'-yl-)-2-(4'-chlorphenoxy)-essigsäureanilid,
2-(1',2',4'-Triazol-1'-yl-)-2-(4'-phenylphenoxy)-essigsäure-p-fluoranilid,
2-(1',2',4'-Triazol-1'-yl)-2-(4'(3''-fluorphenylazo)-phenoxy)-essigsäureanilid oder
2-(1',2',4'-Triazolyl-l'-yl)-2-(4'-chlorphenoxy)-essigsäure-α-naphthylamid.

**Claims for the contracting states BE, CH, DE, FR, GB, IT, LU, NL, SE**

1. 1,2,4-Triazol-1-yl compounds of the general formula I

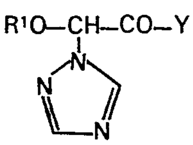
I,

where
$R^1$ denotes phenyl substituted by clyclohexyl, phenyl, 1 to 3 halogen atoms or alkyl of 1 to 4 carbon atoms, and

Y denotes $OR^2$ or 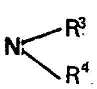 where

$R^2$ denotes alkynyl of 3 to 15 carbon atoms,
$R^3$ denotes hydrogen, alkyl of 1 to 20 carbon atoms,
alkenyl of 3 to 15 carbon atoms, phenyl or cyanoethyl, and
$R^4$ denotes phenyl or benzyl optionally substituted by 1 or 2 halogen atoms, trifluoromethyl or alkyl of 1 to 4 carbon atoms, or alkenyl or alkynyl of 3 to 15 carbon carbon atoms, phenylethyl, naphthyl or cyanoethyl,
and their plant-compatible salts and metal complexes.

2. A process for the manufacture of the compounds as claimed in claim 1, characterized in that

a) a compound of the formula II

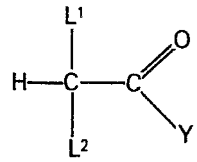
(II)

where Y has the meanings given in claim 1 and $L^1$ and $L^2$ are identical or different and are nucleophilically displaceable leaving groups, is reacted with a compound of the formula III

$R^1OH$ III,

where $R^1$ has the meanings given in claim 1, or a salt thereof, and with 1,2,4-triazole, or a salt thereof, in the presence or absence of a solvent or diluent and/or of an inorganic or organic base and in the presence or absence of a reaction accelerator at from 0° to 180 °C, or

b) a compound of the formula IV

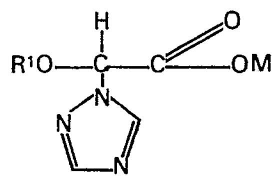
(IV)

where $R^1$ has the meanings given in claim 1 and M denotes hydrogen, one equivalent of a metal cation or a substituted or unsubstituted ammonium ion, is reacted with a compound of the formula V

HY V,

where Y has the meanings given in claim 1, after or with simultaneous addition of reagents suitable for derivatizing acids, in the presence or absence of a solvent or diluent and/or of an inorganic or organic base and in the presence or absence of a reaction accelerator at from −20° to +180 °C, or

c) a compound of the formula IV is reacted with a compound of the formula VI

$L^3Y$ VI,

where Y has the meanings given in claim 1 and $L^3$ is a nucleophilically displaceable leaving group, in the presence or absence of a solvent or diluent and/or an acid binder and in the presence or absence of a reaction accelerator at from −20° to +180 °C, or

d) a compound of the formula VII

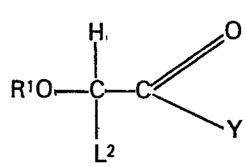
(VII)

where $R^1$, Y and $L^2$ have the above meanings, is

reacted with 1,2,4-triazole in the presence or absence of a solvent or diluent and/or an inorganic or organic base and in the presence or absence of a reaction accelerator at from −20° to +180 °C, and - if desired - the compounds obtained according to a) to d) are converted into their plant-compatible salts and metal complexes.

3. A fungicide containing at least one compound as claimed in claim 1.

4. A fungicide containing at least one compound as claimed in claim 1 and a solid or liquid carrier.

5. A process for combating fungi, characterized in that at least one 1,2,4-triazol-1-yl compound as claimed in claim 1 is allowed to act thereon.

6. A process for producing fungicides, characterized in that 1,2,4-triazol-1-yl compounds of the formula I as claimed in claim 1 are mixed with solid or liquid carriers.

7. A process for the preventive control of fungi, characterized in that at least one fungicide as claimed in claim 1 is allowed to act on areas, plants or seed threatened by fungus attack.

8. A compound as claimed in claim 1, namely
2-(1′,2′,4′-triazol-1′-yl)-2-(4′-chlorophenoxy)-acetic acid anilide,
2-(1′,2′,4′-triazol-1′-yl)-2-(4′-phenylphenoxy)-acetic acid p-fluoroanilide,
2-(1′,2′,4′-triazol-1′-yl)-2-(4′(3″-fluorophenylazo)-phenoxy)-acetic acid anilide or
2-(1′,2′,4′-triazol-1′-yl)-2-(4′-chlorophenoxy)-acetic acid α-naphthylamide.

9. A fungicide containing a compound as claimed in claim 8.

**Claims for the contracting state AT**

1. A fungicide containing a 1,2,4-triazol-1-yl compound of the general formula I

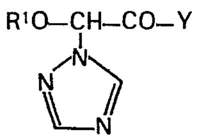
I,

where
$R^1$ denotes phenyl substituted by cyclohexyl, phenyl, 1 to 3 halogen atoms or alkyl of 1 to 4 carbon atoms, and

Y denotes $OR^2$ or 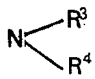 where,

$R^2$ denotes alkynyl of 3 to 15 carbon atoms,
$R^3$ denotes hydrogen, alkyl of 1 to 20 carbon atoms, alkenyl of 3 to 15 carbon atoms, phenyl or cyanoethyl, and
$R^4$ denotes phenyl or benzyl optionally substituted by 1 or 2 halogen atoms, trifluoromethyl or alkyl of 1 to 4 carbon atoms, or alkenyl or alkynyl or 3 to 15 carbon atoms, phenylethyl, naphthyl or cyanoethyl,
and its plant-compatible salts and metal complexes.

2. A process for the manufacture of the compounds as defined in claim 1, characterized in that

a) a compound of the formula II

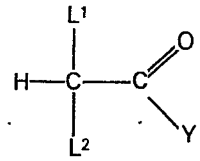
(II)

where Y has the meanings given in claim 1 and $L^1$ and $L^2$ are identical or different and are nucleophilically displaceable leaving groups, is reacted with a compound of formula III

$R^1OH$ III,

where $R^1$ has the meanings given in claim 1, or a salt thereof, and with 1,2,4-triazole, or a salt thereof, in the presence or absence of a solvent or diluent and/or of an inorganic or organic base and in the presence or absence of a reaction accelerator at from 0° to 180 °C, or

b) a compound of the formula IV

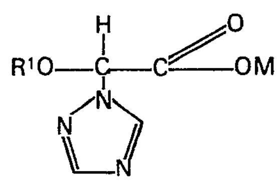
(IV)

where $R^1$ has the meanings given in claim 1 and M denotes hydrogen, one equivalent of a metal cation or a substituted or unsubstituted ammonium ion, is reacted with a compound of the formula V

HY V,

where Y has the meanings given in claim 1, after or with simultaneous addition of reagents suitable for derivatizing acids, in the presence or absence of a solvent or diluent and/or of an inorganic or organic base and in the presence or absence of a reaction accelerator at from −20° to +180 °C, or

c) a compound of the formula IV is reacted with a compound of the formula VI

$L^3Y$ VI,

where Y has the meanings given in claim 1 and $L^3$ is a nucleophilically displaceable leaving group, in the presence or absence of a solvent or diluent and/or an acid binder and in the presence or absence of a reaction accelerator at from −20° to +180 °C, or

d) a compound of the formula VII

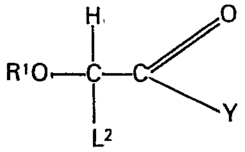

(VII)

where $R^1$, Y and $L^2$ have the above meanings, is reacted with 1,2,4-triazole in the presence or absence of a solvent or diluent and/or an inorganic or organic base and in the presence or absence of a reaction accelerator at from −20° to +180 °C, and – if desired – the compounds obtained acording to a) to d) are converted into their plant-compatible salts and metal complexes.

3. A fungicide containing at least one compound as defined in claim 1 and a solid or liquid carrier.

4. A process for combating fungi, characterized in that at least one 1,2,4-triazol-1-yl compound as defined in claim 1 is allowed to act thereon.

5. A process for producing fungicides, characterized in that 1,2,4-triazol-1-yl compounds as defined in claim 1 are mixed with solid or liquid carriers.

6. A process for the preventive control of fungi, characterized in that at least one fungicide as claimed in claim 1 is allowed to act on areas, plants or seed threatened by fungus attack.

7. A fungicide as claimed in claim 1 containing
2-(1′,2′,4′-triazol-1′-yl)-2-(4′-chlorophenoxy)-acetic acid anilide,
2-(1′,2′,4′-triazol-1′-yl)-2-(4′-phenylphenoxy)-acetic acid p-fluoroanilide,
2-(1′,2′,4′-triazol-1′-yl)-2-(4′(3″-fluorophenylazo)-phenoxy)-acetic acid anilide or
2-(1′,2′,4′-triazol-1′-yl)-2-(4′-chlorophenoxy)-acetic acid α-naphthylamide.

**Revendications pour les états contractants BE, CH, DE, FR, GB, IT, LU, NL, SE**

1. Composés 1,2,4-triazol-1-yliques de la formule générale I

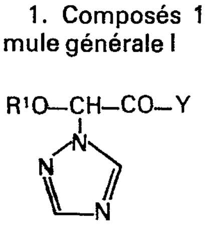

I,

dans laquelle
$R^1$ désigne un groupe phényle substitué par un groupe cyclohexyle ou phényle ou par 1 à 3 atomes d'halogène ou radicaux alcoyle en $C_1$ à $C_4$;

Y représente un groupe $-OR^2$ ou 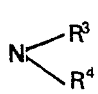 , où

$R^2$ désigne un radical alcynyle en $C_3$ à $C_{15}$,
$R^3$ un atome d'hydrogène, un radical alcoyle en $C_1$ à $C_{20}$ ou alcényle en $C_3$ à $C_{15}$, un groupe phényle ou cyanéthyle et
$R^4$ un groupe phényle ou benzyle éventuellement substitué par 1 ou 2 atomes d'halogène, radicaux trifluorométhyle ou alcoyle en $C_1$ à $C_4$, un radical alcényle ou alcynyle en $C_3$ à $C_{15}$ ou un groupe phényl-éthyle, naphtyle ou cyan-éthyle,

ainsi que leurs sels et complexes de métaux tolérés par les plantes.

2. Procédé de préparation des composés suivant la revendication 1, caractérisé en ce que l'on fait réagir:

a) des composés de la formule II

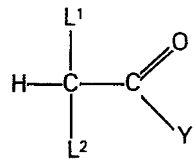

(II)

dans laquelle Y possède les significations définies dans la revendication 1 et $L^1$ et $L^2$, qui peuvent être identiques ou différents, représentent des groupes pouvant être déplacés par des nucléophiles, avec un composé de la formule III

$R^1OH$ (III),

dans laquelle $R^1$ possède les significations définies dans la revendication 1, ou un sel d'un tel composé, et le 1,2,4-triazole ou un de ses sels à des températures comprises entre 0 et 180 °C, éventuellement en présence d'un solvant ou diluant et(ou) d'une base organique ou inorganique, le cas échéant avec addition d'un accélérateur de la réaction; ou

b) des composés de la formule IV

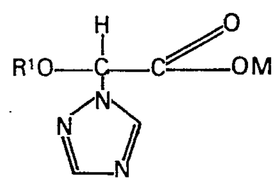

(IV)

dans laquelle $R^1$ possède les significations définies dans la revendication 1 et M désigne un atome d'hydrogène, un équivalent d'un cation de métal ou un ion ammonium éventuellement substitué, avec un composé de la formule V

HY (V),

dans laquelle Y possède la signification définie dans la revendication 1, avec addition préalable ou simultanée de réactifs provoquant la formation de dérivés d'acides, à des températures comprises entre −20 et +180 °C, éventuellement en présence d'un solvant ou diluant et(ou) de bases organiques ou inorganiques, le cas échéant en présence d'un accélérateur de la réaction; ou

c) un composé de la formule IV avec un composé de la formule VI

$L^3Y$ (VI),

dans laquelle Y possède de la signification définie dans la revendication 1 et $L^3$ représente un groupe pouvant être remplacé par un nucléophile, à des températures comprises entre −20 et +180 °C, éventuellement en présence d'un solvant ou diluant et(ou) d'un fixateur d'acide, le cas échéant en présence d'un accélérateur de la réaction; ou

d) des composés de la formule VII

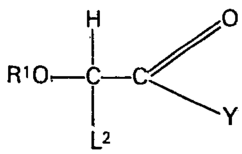

(VII)

dans laquelle $R^1$, Y et $L^2$ possèdent la signification définie plus haut, avec le 1,2,4-triazole à des températures comprises entre −20 et 180 °C, éventuellement en présence d'un solvant ou diluant et(ou) d'une base organique ou inorganique et le cas échéant en présence d'un accélérateur de la réaction,
les composés obtenus sub a) à d) étant, si on le désire, transformés en leurs sels et complexes de métaux tolérés par les plantes.

3. Composition fongicide, contenant au moins un composé suivant la revendication 1.

4. Composition fongicide, contenant au moins un composé suivant la revendication 1 et un véhicule solide ou liquide.

5. Procédé pour combattre les champignons, caractérisé en ce que l'on expose ceux-ci à l'action d'au moins un dérivé 1,2,4-triazol-1-ylique suivant la revendication 1.

6. Procédé de préparation de compositions fongicides, caractérisé en ce que l'on mélange des dérivés 1,2,4-triazol-1-yliques suivant la revendication 1 à des véhicules ou supports solides ou liquides.

7. Procédé de lutte préventive contre les champignons, caractérisé en ce que l'on fait agir au moins un fongicide suivant la revendication 1 sur les surfaces, plantes ou semences risquant d'être attaquées par des champignons.

8. Composé suivant la revendication 1, notamment:

le 2-(1′,2′,4′-triazol-1′-yl)-2-(4′-chlorophénoxy)-acétanilide,
le 2-(1′,2′,4′-triazol-1′-yl)-2-(4′-phénylphénoxy)-acétyl-p-fluoranilide,
le 2-(1′,2′,4′-triazol-1′-yl)-2-(4′-(3″-fluorophénylazo)-phénoxy-acétanilide,
le 2-(1′,2′,4′-triazol-1′-yl)-2-(4′-chlorophénoxy)-acétyl-α-naphtylamide.

9. Composition fongicide, contenant un composé suivant la revendication 8.

**Revendications pour l'état contractant AT**

1. Composition fongicide, contenant un dérivé 1,2,4-triazol-1-ylique de la formule générale I

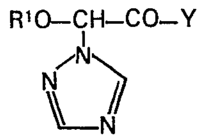

I,

dans laquelle
$R^1$ désigne un groupe phényle substitué par un groupe cyclohexyle ou phényle ou par 1 à 3 atomes d'halogène ou radicaux alcoyle en $C_1$ à $C_4$;

Y représente un groupe-$OR^2$ ou 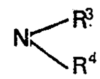 où

$R^2$ désigne un radical alcynyle en $C_3$ à $C_{15}$,
$R^3$ un atome d'hydrogène, un radical alcoyle en $C_1$ à $C_{20}$ ou alcényle en $C_3$ à $C_{15}$, un groupe phényle ou cyanéthyle et
$R^4$ un groupe phényle ou benzyle éventuellement substitué par 1 ou 2 atomes d'halogène, radicaux trifluorométhyle ou alcoyle en $C_1$ à $C_{41}$, un radical alcényle ou alcynyle en $C_3$ à $C_{15}$ ou un groupe phényl-éthyle, naphtyle ou cyan-éthyle,
ou un de ses sels ou complexes de métaux tolérés par les plantes.

2. Procédé de préparation des composés définis dans la revendication 1, caractérisé en ce que l'on fait réagir:

a) des composés de la formule II

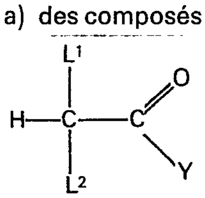

(II)

dans laquelle Y possède les significations définies dans la revendication 1 $L^1$ et $L^2$, qui peuvent être identiques ou différents, représentent des groupes pouvant être déplacés par des nucléophiles, avec un composé de la formule III

$R^1OH$ (III),

dans laquelle $R^1$ possède les significations définies dans la revendication 1, ou un sel d'un tel composé, et le 1,2,4-triazole ou un de ses sels à des températures comprises entre 0 et 180 °C, éventuellement en présence d'un solvant ou diluant et(ou) d'une base organique ou inorganique, le cas échéant avec addition d'un accélérateur de la réaction; ou

b) des composés de la formule IV

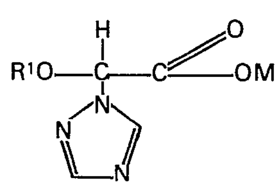

(IV)

dans laquelle $R^1$ possède les significations définies dans la revendication 1 et M désigne un atome d'hydrogène, un équivalent d'un cation de métal ou un ion ammonium éventuellement substitué, avec un composé de la formule V

HY (V),

dans laquelle Y possède la signification définie dans la revendication 1, avec addition préalable ou simultanée de réactifs provoquant la formation de dérivés d'acides, à des températures comprises entre −20 et +180 °C, éventuellement en présence d'un solvant ou diluant et(ou) de bases organiques ou inorganiques, le cas échéant en présence d'un accélérateur de la réaction; ou

c) un composé de la formule IV avec un composé de la formule VI

$L^3Y$ (VI),

dans laquelle Y possède la signification définie dans la revendication 1 et $L^3$ représente un groupe pouvant être remplacé par un nucléophile, à des températures comprises entre −20 et +180 °C, éventuellement en présence d'un solvant ou diluant et(ou) d'un fixateur d'acide, le cas échéant en présence d'un accélérateur de la réaction; ou

d) des composés de la formule VII

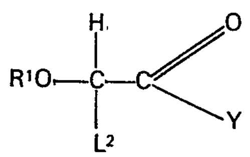

(VII)

dans laquelle $R^1$, Y et $L^2$ possèdent la signification définie plus haut, avec le 1,2,4-triazole à des températures comprises entre −20 et 180 °C, éventuellement en présence d'un solvant ou diluant et(ou) d'une base organique ou inorganique et le cas échéant en présence d'un accélérateur de la réaction,

les composés obtenus sub a) à d) étant, si on le désire, transformés en leurs sels et complexes de métaux tolérés par les plantes.

3. Composition fongicide, contenant au moins un composé suivant la revendication 1 et un véhicule solide ou liquide.

4. Procédé pour combattre les champignons, caractérisé en ce que l'on expose ceux-ci à l'action d'au moins un dérivé 1,2,4-triazol-1-ylique suivant la revendication 1.

5. Procédé de préparation de compositions fongicides, caractérisé en ce que l'on mélange des dérivés 1,2,4-triazol-1-yliques suivant la revendication 1 à des véhicules ou supports solides ou liquides.

6. Procédé de lutte préventive contre les champignons, caractérisé en ce que l'on fait agir au moins un fongicide suivant la revendication 1 sur les surfaces, plantes ou semences risquant d'être attaquées par des champignons.

7. Composé suivant la revendication 1, notamment:

le 2-(1′,2′,4′-triazol-1′-yl)-2-(4′-chlorophénoxy)-acétanilide,
le 2-(1′,2′,4′-triazol-1′-yl)-2-(4′-phénylphénoxy)-acétyl-p-fluoranilide,
le 2-(1′,2′,4′-triazol-1′-yl)-2-(4′-(3″-fluorophénylazo)-phénoxy-acétanilide,
le 2-(1′,2′,4′-triazol-1′-yl)-2-(4′-chlorophénoxy)-acétyl-α-naphtylamide.